# EUROPEAN PATENT APPLICATION

(11) **EP 4 570 885 A1**
(43) Date of publication of application: **18.06.2025**
(21) Application number: 23307222.2
(22) Date of filing: 15.12.2023
(51) Int. Cl.: C10L 3/08, C07C 1/12

(54) **AN INSTALLATION ADAPTED FOR PROCESSING A CO2 RICH GAS AND WASTES CONTAINING ORGANIC MATTER, AND FOR PRODUCING A METHANE RICH GAS USING RENEWABLE ENERGY**

(71) Applicant: Technip Energies France, 92741 Nanterre Cedex (FR); Commissariat à l'Energie Atomique et aux Energies Alternatives, 75015 Paris (FR)
(72) Inventor: MILLERAND, Xavier, 92741 NANTERRE CEDEX (FR); BARRON, Estefany, 92741 NANTERRE CEDEX (FR); LANESE, Valeriano, 92741 NANTERRE CEDEX (FR); BEDEL, Laurent, 38054 GRENOBLE (FR); DUCROS, Frédéric, 38054 GRENOBLE (FR); VALIN, Sylvie, 38054 GRENOBLE (FR); GEFFRAYE, Geneviève, 38054 GRENOBLE (FR)
(74) Representative: Edson, Russell Gregory

(57) **Abstract**

An installation (10) comprising:
- an electrolysis unit (24) for receiving power (26) and steam (28), and for producing a hydrogen rich gas (30),
- a gasification unit (34) for receiving wastes (16) containing organic matter, and an oxygen rich gas (32B), and for producing syngas (38),
- a catalytic methanation unit (44) for receiving a CO2 rich gas (14), said hydrogen rich gas and said syngas, and for producing a methane rich gas (18),
- a steam generation unit (50) for receiving heat (54) from the catalytic methanation unit (44), and producing said steam.

The installation is adapted for switching at least between a first operating mode, in which said power is intended to be fully renewable, and a second operating mode, in which said power is at a lower level and is intended to be renewable and/or from an electricity grid (58).

## Description

### Field of the disclosure

The present disclosure deals with an installation adapted for processing a CO2 rich gas and for producing a methane rich gas, the installation comprising a methanation unit adapted for receiving said CO2 rich gas, and for producing said methane rich gas, wherein the installation is adapted for using power intended to be fully provided by one or several renewable energy source(s), when such power is available.

The disclosure also deals with a corresponding method.

The CO2 rich gas for example comes from a biogas unit also producing wastes containing organic matter that are intended to be discarded.

### Description of related art

Biomethane production from biowaste, for example using the anaerobic digestion, is a well-known process. The biowaste is fed to a digestor that produces biogas, digestate and discarded waste. The biogas, mainly containing methane and CO2, is processed to produce biomethane. However, the separated biogenic CO2 is usually discharged to the atmosphere, while the discarded waste is sent to landfill. Besides, the biomethane production yield is low, since 40-50 vol.% of the biogas is CO2.

CO and CO2 methanation is a mature process that converts carbon and hydrogen atoms to produce Synthetic Natural Gas (SNG). The methanation process can take place chemically or biologically, nevertheless chemical catalytic methanation is the main method in industrial applications. Reaction takes place at temperatures around 200 to 750°C typically using a nickel catalyst. The most developed methanation processes use syngas (a mixture containing mostly CO and H2) as feedstock, originating from gasification of solid or liquid feedstocks (coal, biomass, pyrolysis oils). In this context, many processes such as Lurgi, HICOM, and TREMP have been developed using adiabatic fixed bed reactors. These processes however aim at the continuous treatment of large gas flows, and are not compatible with renewable energy with fluctuating availability.

An aim is to provide an installation able to convert a CO2 rich gas, for example coming from a biogas unit, into a methane rich gas in a thermally efficient way and using renewable energy with fluctuating availability.

### Summary

To this end, the disclosure proposes an installation adapted for receiving a CO2 rich gas and wastes containing organic matter, and for producing a methane rich gas, the installation comprising:
- an electrolysis unit adapted for receiving power and steam, and for producing a hydrogen rich gas,
- a gasification unit adapted for receiving the wastes, an oxygen rich gas and boiler feed water, and for producing syngas,
- a catalytic methanation unit adapted for receiving said CO2 rich gas, said hydrogen rich gas and said syngas, and for producing said methane rich gas, and
- a steam generation unit adapted for receiving water and heat, and for producing said steam received by the electrolysis unit and said boiler feed water, said heat coming from the catalytic methanation unit,
wherein the installation is adapted for switching at least between:
- a first operating mode, in which said power is at a first level and is intended to be fully provided by one or several renewable energy source(s), and
- a second operating mode, in which said power is at a second level, lower than the first level, and said power is intended to be provided by said renewable energy source(s) and/or by an electricity grid.

In other embodiments, the installation comprises one or several of the following features, taken in isolation or any technically feasible combination:
- the electrolysis unit is adapted for producing an oxygen containing gas, the installation comprising a purification unit adapted for purifying the oxygen containing gas in order to produce at least part of the oxygen rich gas;
- each of the electrolysis unit, the gasification unit and the catalytic methanation unit is adapted for running at a reduced capacity in the second operating mode compared with the first operating mode;
- the installation comprises a water purification unit adapted for receiving flows of recycled water the gasification unit and the catalytic methanation unit, and for producing at least part of said water received by the steam generation unit;
- the installation comprises a pretreatment unit adapted for drying and grinding said wastes before said wastes enter the gasification unit;
- the electrolysis unit comprises solid oxide electrolyzer cells;
- the solid oxide electrolyzer cells are configured for operating at atmospheric pressure and at a temperature between 750°C and 850°C;
- the methanation unit comprises a shell and tube heat exchanger catalytic reactor;
- the catalytic reactor contains tubes, and a catalytic powder bed confined within the tubes;
- the installation comprises said renewable energy source(s), wherein: in the first operating mode, said power is fully provided the renewable energy source(s); and in the second operating mode, said power is provided by said renewable energy source(s) and/or is intended to be provided by the electricity grid;
- the installation comprises a biogas unit adapted for producing a methane rich biogas, said CO2 rich gas, and said wastes;
- the biogas unit comprises a methanisation unit adapted for producing a first biogas flow by anaerobic digestion, a gas holder adapted for storing the first biogas flow and an upgrading system adapted for receiving a second biogas flow coming from the gas holder, and for producing the methane rich biogas and said CO2 rich gas; and
- the biogas unit is adapted for producing a CO2 rich exhaust, the installation being adapted for using at least part of the CO2 rich exhaust in order to form the CO2 rich gas; and in the second operating mode, at least a part of the CO2 rich exhaust forms an exhaust.

The disclosure also proposes a method of producing a methane rich gas using a CO2 rich gas and wastes containing organic matter, comprising the following steps:
- providing an installation as described above,
- receiving power and steam in the electrolysis unit, and producing a hydrogen rich gas,
- receiving the wastes, an oxygen rich gas and boiler feed water in the gasification unit, and producing syngas,
- receiving said CO2 rich gas, said hydrogen rich gas and said syngas in the catalytic methanation unit, and producing said methane rich gas,
- in the steam generation unit, receiving water and heat, and producing said steam received by the electrolysis unit, said heat coming from the catalytic methanation unit, and
- switching the installation at least between: a first operating mode, in which said power is at a first level and is fully provided by said one or several renewable energy source(s); and a second operating mode, in which said power is at a second level, lower than the first level, and said power is provided by said renewable energy source(s) and/or by the electricity grid.

### Brief description of the drawings

The disclosure and its advantages will be better understood upon reading the following description, given solely by way of example and with reference to the appended drawings, in which:
- Figure 1 is a diagram showing an installation according to the disclosure, the installation being in a first operating mode, using renewable electricity and no electricity from the grid, and
- Figure 2 is diagram showing the installation shown in Figure 1, the installation being in a second operating mode, using less or no renewable electricity, and possibly some electricity from the grid.

### Detailed description

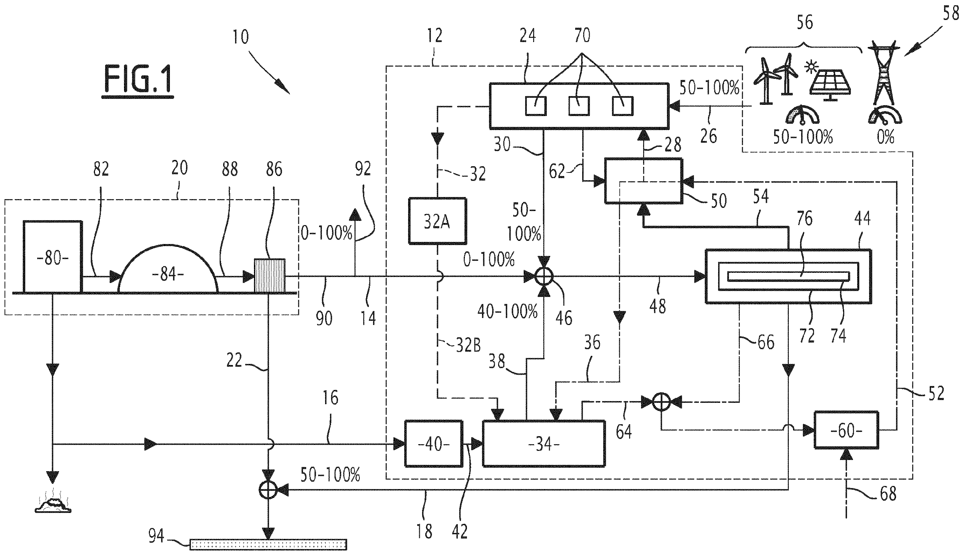

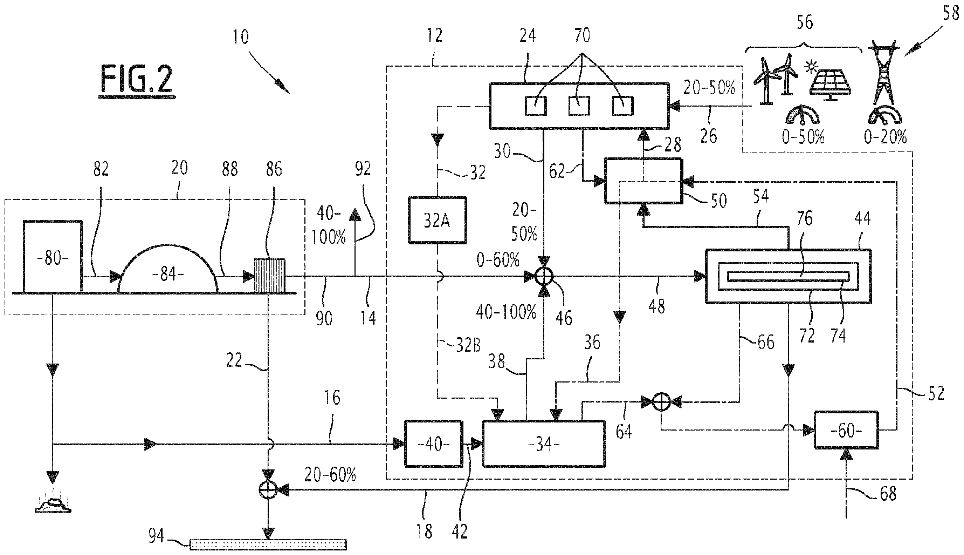

An installation 10 according to the disclosure will now be described with reference to Figures 1 and 2.

The installation 10 comprise a portion 12 adapted for processing a CO2 rich gas 14 and wastes 16 containing organic matter, and for producing a methane rich gas 18.

In the example, the installation 10 comprises a biogas unit 20 adapted for producing a methane rich biogas 22, said CO2 rich gas 14, and said wastes 16. In other words, in the example, the CO2 rich gas 14 and the wastes 16 are self-produced.

As a variant (not shown), the installation 10 does not comprises a biogas unit, and the CO2 rich gas 14 and the wastes 16 come from outside the installation. For example, the CO2 rich gas 14 and the wastes 16 may come from a water treatment plant (not shown), which can be part of the installation 10 or be outside of the installation.

In the present document, "CO2 rich gas", or more generally a gas rich in a molecule or a group of molecules, for example means a content of 90 vol.% or more of the molecule(s).

The installation 10 comprises an electrolysis unit 24 adapted for receiving power 26 and steam 28, and for producing a hydrogen rich gas 30 and advantageously an oxygen containing gas 32, for example containing about 30vol.% of oxygen.

The installation 10 comprises a gasification unit 34 adapted for receiving the wastes 16, an oxygen rich gas 32B and boiler feed water 36, and for producing syngas 38.

The installation 10 for example comprises a pretreatment unit 40 adapted for drying and grinding said wastes 16 in order to obtain dried particles 42 received in the gasification unit 34.

As variants (not shown), the installation 10 does not comprise the pretreatment unit 40, or the pretreatment unit 40 is of a different type, or is integrated within the gasification unit 34.

The installation 10 advantageously comprises a purification unit 32A adapted for purifying the oxygen containing gas 32 and for producing the oxygen rich gas 32B.

As a variant (not shown), the installation 10 does not comprise the purification unit 32A, and the oxygen rich gas 32B comes from a source (not shown) outside the installation 10.

In a particular embodiment, the oxygen rich gas 32B may be partly produced by the purification unit 32A and partly come from said outside source (not shown).

The installation 10 comprises a catalytic methanation unit 44 adapted for receiving the CO2 rich gas 14, the hydrogen rich gas 30 and the syngas 38, and for producing said methane rich gas 18. For example, a mixing system 46 of the installation is adapted for mixing the CO2 rich gas 14, the hydrogen rich gas 30 and the syngas 38 in order to produce a feed 48 for the catalytic methanation unit 44.

The installation 10 comprises a steam generation unit 50 adapted for receiving water 52 and heat 54, and for producing the steam 28 received by the electrolysis unit and the boiler feed water 36 received by the gasification unit, said heat coming from the catalytic methanation unit 44. In other words, there is heat integration between the catalytic methanation unit 44 and the steam generation unit 50.

In a particular embodiment, all the heat received by the steam generation unit 50 comes from the catalytic methanation unit 44 only.

The installation 10 is adapted for switching at least between a first operating mode (Figure 1), and a second operating mode (Figure 2).

In the first operating mode, the power 26 is at a first level, for example 50% to 100% of a maximum power, and is fully provided by one or several renewable energy source(s) 56, which in the example are part of the installation 10.

"Maximum power" or "maximum flow" is to be understood as a maximum value during normal operation of the installation 10.

In the second operating mode, the power 26 is at a second level, lower than the first level, for example 20% to 50% of the maximum power, and the power 26 is provided by said renewable energy source(s) 56 and/or by an outside electricity grid 58. For example, the renewable energy source(s) provide(s) 0% to 50% of the maximum power, and the grid provides 0% to 20% of the maximum power.

In a particular embodiment, in the second operating mode, the power 26 is exclusively provided by the electricity grid 58.

Advantageously, each of the electrolysis unit 24, the gasification unit 34 and the catalytic methanation unit 44 is adapted for running at a reduced capacity in the second operating mode compared with the first operating mode.

For example, the flowrate of the hydrogen rich gas 30 is between 50% and 100% of a maximum flowrate in the first operating flowrate mode, and between 20% and 50% of the maximum flowrate in the second operating mode.

For example, the flowrate of the CO2 rich gas 14 is between 0% and 100% of a maximum flowrate in the first operating mode, and between 0% and 60% of the maximum flowrate in the second operating mode.

"Between A% and B%" is to be understood in the present document as larger than A% and smaller than B%.

For example, the flowrate of syngas 38 is between 40% and 100% of a maximum flowrate in the first operating mode, and in the second operating mode.

For example, the flowrate of the methane rich gas 18 is between 50% and 100% of a maximum flowrate in the first operating mode, and between 20% and 60% of the maximum flowrate in the second operating mode.

Advantageously, the installation 10 comprises a water purification unit 60 adapted for receiving flows of recycled water 64, 66 respectively from the gasification unit 34 and the catalytic methanation unit 44, and for producing at least part of said water 52 received by the steam generation unit 50.

The electrolysis unit 24, gasification unit 34, catalytic methanation unit 44 and biogas unit 20 are known in themselves and will not be described in detail.

In a particular embodiment, the water purification unit 60 is adapted for receiving extra water 68.

The renewable energy source(s) 56 for example include a windmill, solar and/or hydraulic facility.

The electrolysis unit 24 advantageously comprises solid oxide electrolyzer cells 70.

The solid oxide electrolyzer cells 70 are for example configured for operating at atmospheric pressure and at a temperature between 750°C and 850°C.

The electrolysis unit 24 is for example adapted for producing a hot mixture (not shown) containing hydrogen and steam, and for cooling down this mixture in order to obtain the hydrogen rich gas 30 and a stream of water 62 that is advantageously sent to the steam generation unit 50.

The methanation unit 44 advantageously comprises a shell and tube heat exchanger catalytic reactor 72.

The catalytic reactor 72 for example comprises tubes 74, and a catalytic powder bed 76 confined within the tubes 74. Such a catalytic reactor 72 is for example described in FR-A-1 913 378.

The biogas unit 20 for example comprises a methanisation unit 80 adapted for producing a first biogas flow 82 by anaerobic digestion, a gas holder 84 adapted for storing the first biogas flow, and an upgrading system 86 adapted for receiving a second biogas flow 88 coming from the gas holder, and for producing the methane rich biogas 22 and said CO2 rich gas 14.

The biogas unit 20 is adapted for producing a CO2 rich exhaust 90, the installation 10 being adapted for using at least part of the CO2 rich exhaust 90 in order to form the CO2 rich gas 14. In other words, the CO2 rich gas 14 comes from, or is the CO2 rich exhaust 90.

In the second operating mode, at least a part of CO2 rich exhaust 90 forms an exhaust 92 which is not used to form the CO2 rich gas 14.

For example, the flowrate of the exhaust 92 is between 0% and 100% of a maximum flowrate in the first operating mode, and between 40% and 100% of the maximum flowrate in the second operating mode.

The operation of the installation 10 stems from its structure and will now be described in order to illustrate a method according to the disclosure.

For example the installation 10 is initially in the first operating mode.

The biogas unit 20 produces the wastes 16, and the CO2 rich exhaust 90 from which the CO2 rich gas 14 is obtained.

The flowrate of the CO2 rich gas 14 is for example between 0% and 100% of its maximum flowrate.

The flowrate of the exhaust 92 is for example between 0% and 100% of its maximum flowrate. Preferably, all of the CO2 rich exhaust 90 is used to form the CO2 rich gas 14, and there is no exhaust (0% of its maximum flowrate).

The power 26 and steam 28 are received in the electrolysis unit 24, which produces the hydrogen rich gas 30 and the oxygen containing gas 32, which is in the example purified by the purification unit 32A into the oxygen rich gas 32B.

The power 26 is at the first level, for example from 50% to 100% of the maximum power, and is fully provided by the renewable energy source(s) 56. The flowrate of the hydrogen rich gas 30 is between 50% and 100% of the maximum flowrate.

Advantageously, the pretreatment unit 40 dries and grinds the wastes 16.

The wastes 16, the oxygen rich gas 32 and boiler feed water 36 are received in the gasification unit 34, which produces the syngas 38.

The flowrate of the syngas 38 is for example between 40% and 100% of its maximum flowrate.

The CO2 rich gas 14, the hydrogen rich gas 30 and the syngas 38 are received in the catalytic methanation unit 44, which produces the methane rich gas 18, at a flowrate for example between 50% and 100% of the maximum flowrate.

The steam generation unit 50 receives the water 52, and the heat 54 from the catalytic methanation unit 44, and produces the steam 28 received by the electrolysis unit 24 and the boiler feed water 36 received by the gasification unit 34.

The methane rich gas 18 and the methane rich biogas 22 are for example merged and injected in a gas grid 94.

Then the installation 10 is switched from the first operating mode to the second operating mode.

The power 25 is at the second level, for example from 20% to 50% of the maximum power, and is for example fully provided by the renewable energy source(s) 56. The flowrate of the hydrogen rich gas 30 is between 20% and 50% of the maximum flowrate.

The flowrate of the CO2 rich gas 14 is for example between 0% and 60% of its maximum flowrate.

The flowrate of the exhaust 92 is for example between 40% and 100% of its maximum flowrate.

The flowrate of syngas 38 is for example between 40% and 100% of its maximum flowrate.

The flowrate of the methane rich gas 18 is for example between 20% and 60% of its maximum flowrate.

Thanks to the above described features, the installation 10 is able to convert the CO2 rich gas 14, for example coming from the biogas unit 20, into a methane rich gas 18 in a thermally efficient way and using renewable energy with fluctuating availability.

The CO2 rich exhaust 90 and the wastes 16 from the biogas unit 20, instead of being discharged to the atmosphere and sent to landfill respectively, are at least partly processed in the portion 12 of the installation 10, which increases the yield of transformation into methane.

The methane production is increased by 150% compared to that of the biogas unit 20 considered alone.

CO2 emissions to the atmosphere and discarded wastes are reduced.

The hydrogen rich gas production benefits from a reduced electrolyser energy consumption, as thermal energy from the methanation unit 44 is recovered.

The renewable energy source intermittency is managed using the flexibility of the installation 10, allowing to operate at least in the first operating mode (maximum capacity, when renewable energy is available) and in the second operating mode (turn down capacity when renewable energy source is not available or less available).

The installation 10 enables water circularity. Contaminated water, after purification, is reused to produce the steam fed to the electrolysis unit 24. The water consumption of the installation 10 is greatly reduced.

The intermittency of renewable energy may be managed without considering any energy storage. Advantageously, each electron is directly converted into gas that may be continuously injected into the grid 94.

## Claims

1. An installation (10) adapted for processing a CO2 rich gas (14) and wastes (16) containing organic matter, and for producing a methane rich gas (18), the installation (10) comprising:
- an electrolysis unit (24) adapted for receiving power (26) and steam (28), and for producing a hydrogen rich gas (30),
- a gasification unit (34) adapted for receiving the wastes (16), an oxygen rich gas (32B) and boiler feed water (36), and for producing syngas (38),
- a catalytic methanation unit (44) adapted for receiving said CO2 rich gas (14), said hydrogen rich gas (30) and said syngas (38), and for producing said methane rich gas (18), and
- a steam generation unit (50) adapted for receiving water (52) and heat (54), and for producing said steam (28) received by the electrolysis unit (24) and said boiler feed water (36), said heat (54) coming from the catalytic methanation unit (44),
wherein the installation (10) is adapted for switching at least between:
- a first operating mode, in which said power (26) is at a first level and is intended to be fully provided by one or several renewable energy source(s) (56), and
- a second operating mode, in which said power (26) is at a second level, lower than the first level, and said power (26) is intended to be provided by said renewable energy source(s) (56) and/or by an electricity grid (58).

2. The installation (10) according to claim 1, wherein the electrolysis unit (24) is adapted for producing an oxygen containing gas (32), the installation (10) comprising a purification unit (32A) adapted for purifying the oxygen containing gas (32) in order to produce at least part of the oxygen rich gas (32B).

3. The installation (10) according to claim 1 or 2, wherein each of the electrolysis unit (24), the gasification unit (34) and the catalytic methanation unit (44) is adapted for running at a reduced capacity in the second operating mode compared with the first operating mode.

4. The installation (10) according to any one of claims 1 to 3, comprising a water purification unit (60) adapted for receiving flows of recycled water (64, 66) the gasification unit (34) and the catalytic methanation unit (44), and for producing at least part of said water (52) received by the steam generation unit (50).

5. The installation (10) according to any one of claims 1 to 4, comprising a pretreatment unit (40) adapted for drying and grinding said wastes (16) before said wastes (16) enter the gasification unit (34).

6. The installation (10) according to any one of claims 1 to 5, wherein the electrolysis unit (24) comprises solid oxide electrolyzer cells (70).

7. The installation (10) according to claim 6, wherein the solid oxide electrolyzer cells (70) are configured for operating at atmospheric pressure and at a temperature between 750°C and 850°C.

8. The installation (10) according to any one of claims 1 to 7, wherein the methanation unit (44) comprises a shell and tube heat exchanger catalytic reactor (72).

9. The installation (10) according to claim 8, wherein the catalytic reactor (72) contains tubes (74), and a catalytic powder bed (76) confined within the tubes (74).

10. The installation (10) according to any one of claims 1 to 9, comprising said renewable energy source(s) (56), wherein:
- in the first operating mode, said power (26) is fully provided the renewable energy source(s) (56), and
- in the second operating mode, said power (26) is provided by said renewable energy source(s) (56) and/or is intended to be provided by the electricity grid (58).

11. The installation (10) according to any one of claims 1 to 10, comprising a biogas unit (20) adapted for producing a methane rich biogas (22), said CO2 rich gas (14), and said wastes (16).

12. The installation (10) according to claim 11, wherein the biogas unit (20) comprises a methanisation unit (80) adapted for producing a first biogas flow (82) by anaerobic digestion, a gas holder (84) adapted for storing the first biogas flow (82), and an upgrading system (86) adapted for receiving a second biogas flow (88) coming from the gas holder (84), and for producing the methane rich biogas (22) and said CO2 rich gas (14).

13. The installation (10) according to claim 11 or 12, wherein:
- the biogas unit (20) is adapted for producing a CO2 rich exhaust (90), the installation (10) being adapted for using at least part of the CO2 rich exhaust (90) in order to form the CO2 rich gas (14), and
- in the second operating mode, at least a part of the CO2 rich exhaust (90) forms an exhaust (92).

14. A method of producing a methane rich gas (18) using a CO2 rich gas (14) and wastes (16) containing organic matter, comprising the following steps:
- providing an installation (10) as described by any one of claims 1 to 13,
- receiving power (26) and steam (28) in the electrolysis unit (24), and producing a hydrogen rich gas (30),
- receiving the wastes (16), an oxygen rich gas (32B) and boiler feed water (36) in the gasification unit (34), and producing syngas (38),
- receiving said CO2 rich gas (14), said hydrogen rich gas (30) and said syngas (38) in the catalytic methanation unit (44), and producing said methane rich gas (18),
- in the steam generation unit (50), receiving water (52) and heat (54), and producing said steam (28) received by the electrolysis unit (24)), said heat (54) coming from the catalytic methanation unit (44), and
- switching the installation (10) at least between:
- a first operating mode, in which said power (26) is at a first level and is fully provided by said one or several renewable energy source(s) (56), and
- a second operating mode, in which said power (26) is at a second level, lower than the first level, and said power (26) is provided by said renewable energy source(s) (56) and/or by the electricity grid (58).
